**Europäisches Patentamt**

**European Patent Office**

**Office européen des brevets**

(19)

(11) Veröffentlichungsnummer: **0 133 978**
A2

(12)

# EUROPÄISCHE PATENTANMELDUNG

(21) Anmeldenummer: **84108984.0**

(22) Anmeldetag: **28.07.84**

(51) Int. Cl.⁴: **C 12 N 15/00,** C 12 N 13/00, C 12 M 3/00

(30) Priorität: **01.08.83 DE 3327690**

(43) Veröffentlichungstag der Anmeldung: **13.03.85 Patentblatt 85/11**

(84) Benannte Vertragsstaaten: **CH FR GB LI**

(71) Anmelder: **Kernforschungsanlage Jülich Gesellschaft mit beschränkter Haftung, Postfach 1913, D-5170 Jülich (DE)**

(72) Erfinder: **Zimmermann, Ulrich, Prof., Röntgenstrasse 11, D-8700 Würzburg (DE)**
Erfinder: **Küppers, Gottfried, Dr., Tannenweg 16, D-5138 Heinsberg (DE)**

(54) **Verfahren zur Fusion von Zellen.**

(57) Die Erfindung bezieht sich auf ein Verfahren zur Fusion von Zellen, bei dem in einer Zellsuspension im Fusionsraum befindliche Zellen durch Anwendung eines elektrischen Feldes fusioniert werden. Gemäß der Erfindung wird das elektrische Feld mittels eines als Sender ausgebildeten elektrischen Pulsgenerators erzeugt und die elektrische Energie drahtlos auf den Fusionsraum übertragen. Dabei sind zur Aufnahme der elektrischen Energie im Fusionsraum diesen begrenzende Elektroden vorgesehen, die aus einer Substanz bestehen, die eine gegenüber der Zellsuspension im Fusionsraum höhere Leitfähigkeit, mindestens aber $10^{-3}$ S/cm aufweist.

Die Erfindung bezieht sich ferner auf eine Vorrichtung zur Durchführung des Verfahrens. Bei dieser Vorrichtung ist ein Raum zur Aufnahme der Zellsuspension und wenigstens zwei derart angeordnete Elektroden vorgesehen, daß ein zwischen den Elektroden liegender Bereich gebildet wird. In diesem Bereich werden die Zellen dem zwischen den Elektroden ausgebildeten elektrischen Feld ausgesetzt. Gemäß der Erfindung ist die Einrichtung zur Erzeugung des elektrischen Feldes als Sender ausgebildet.

Verfahren zur Fusion von Zellen
_____

Die Erfindung bezieht sich auf ein Verfahren zur Fusion von Zellen, bei dem in einer Zellsuspension im Fusionsraum befindliche Zellen auf einen geringen Abstand zueinander gebracht werden und die Zellen dem Puls eines elektrischen Feldes einer solchen Stärke ausgesetzt werden, daß die Membran der Zellen durchbricht, wodurch bei benachbarten Zellen Störungen in der Membranstruktur verursacht werden, die zur Bildung von Membranbrücken zwischen den benachbarten Zellen und zur Verschmelzung der Zellen führen. Die Erfindung bezieht sich ferner auf eine Vorrichtung zur Durchführung des Verfahrens.

Aus der DE-PS 24 05 119 ist ein Verfahren zur Behandlung von Zellen im elektrischen Feld bekannt, bei dem die Membran von Zellen durch Anwendung eines elektrischen Feldes, dessen Stärke $10^3$ bis $10^5$ V/cm beträgt, durchbrochen wird. Die dabei bewirkte Permeabilitätserhöhung der Zellmembran macht es möglich, Stoffe durch die Membran auszutauschen, ohne daß die Zellen in ihrer Lebensfähigkeit beeinträchtigt sind. Denn die Erhöhung der Permeabilität ist nach dem Austausch der Stoffe in einem einfachen Verfahrensschritt reversibel. Auf diese Weise ist es beispielsweise auch möglich, Gene oder Enzyme in die Zellen einzulagern.

- 2 -

Das aus der vorgenannten Patentschrift bekannte
Verfahren der Erhöhung der Permeabilität der
Zellmembran ist auch zur Fusion von Zellen
einsetzbar.

Zwei Zellen in einer Suspension sollten, wenn
sie sich berühren und ein enger Kontakt zwischen
den Membranen beider Zellen entsteht, miteinander verschmelzen, da die Bausteine in der
Membran beweglich sind. Eine derartige spontane
Verschmelzung (Fusion) von Zellen wird unter
natürlichen Bedingungen jedoch nicht oder nur
äußerst selten beobachtet. Eine bekannte Ausnahme stellt die Befruchtung einer Eizelle
durch eine Samenzelle bei der sexuellen Fortpflanzung dar. Die spontane Fusion wird dagegen
durch die negative Ladung der Phospholipide
und anderer Membrankomponenten behindert. Sie
führt zur Abstoßung der Zellen, wenn sie sich
auf einen geringen Abstand genähert haben.
Zellverschmelzung erfordert aber, daß die beiden
Membranen sich bis auf einen Abstand von weniger
als $10^{-7}$ cm nähern können.

Die mit technischen Mitteln, also auf künstlichem
Wege durchgeführte Fusion von Zellen ist in
einem weiten Anwendungsbereich einsetzbar.
So ist es für die biologisch-medizinische Forschung von großem Interesse, eine große Zahl
von Zellen miteinander zu verschmelzen. Bei
geeigneter Größe der durch Fusion mehrerer
oder gegebenenfalls vieler Zellen - beispielsweise
1000 bis 10000 Blutkörperchen -

- 3 -

entstandenen großen Zellen lassen sich dann Mikroelektroden, Mikrodruckmeßsonden und andere Sensoren ohne irreversible Zerstörung der Membran in die große Zelle einführen. Die Technik, über die Sensoren direkt eine Reihe von Zellen und Membranfunktionen zu erfassen, ist dabei für die klinische Diagnostik, z.B. bei der Früherkennung von Erkrankungen sowie generell für die Grundlagenforschung von Bedeutung.

Die Technik der Fusion von Zellen kann außerdem eingesetzt werden für die Bildung von Hybridzellen durch Verschmelzung von zwei Zellen unterschiedlicher Herkunft, die evolutionsmäßig nicht zu weit voneinander entfernt stehen sollen. Dabei können Zellhybride aus Pflanzenzellen, aus denen wieder ganze Pflanzen gezüchtet werden können oder Zellhybride aus tierischen Zellen, über die monoklonale Antikörper, z.B. gegen Tumore und Leukämie, gewonnen werden können, gebildet werden. Als Beispiel sei genannt die Verschmelzung einer Lymphozytenzelle mit einer Myelomzelle, die besonders aus medizinischer und pharmazeutischer Sicht von großem Interesse ist. Bestimmte Lymphozyten bilden gegen Fremdstoffe im Organismus Antikörper, z.B. gegen ein Fremdeiweiß, das in die Blutbahn injiziert worden ist. Isoliert man die Lymphozyten und fusioniert sie mit einer Tumorzelle, wie der Myelomzelle, so besteht die Chance, daß sich eine sogenannte Hybridomzelle bildet, die die Eigenschaft beider Elternzellen besitzt. Diese Zelle produziert Antikörper, und zwar spezifisch nur gegen den betreffenden Fremdstoff (sogenannte monoklonale Antikörper). Sie ist unsterblich und läßt sich im Gegensatz zu einer normalen ausdifferenzierten Zelle, wie dem Lymphozyten, permanent in Nährmedien vermehren.

Ein Verfahren zur Fusion von Zellen der eingangs bezeichneten Art ist aus J. Membrane Biol. 67, 165 - 182 (1982), Electric Field-Induced Cell-to-Cell Fusion, U. Zimmermann and J. Vienken, bekannt. Bei diesem bekannten Verfahren - dessen Ablauf unter dem Mikroskop beobachtet werden kann - werden Störungen in der Membranstruktur zwischen benachbarten Zellen durch einen elektrischen Durchbruchpuls ausgelöst. Dabei werden - nach den bisherigen Modellvorstellungen - Löcher in der Membrankontaktzone benachbarter Zellen erzeugt, die zu einem zytoplastischen Kontinuum zwischen den beiden Zellen und zur Brückenbildung von Lipiden zwischen den Membranen der benachbarten Zellen führen. Die Lipidmoleküle ordnen sich nicht mehr in ihre ursprüngliche Membran ein. Sobald sich eine Brücke gebildet hat, kommt es aus energetischen Gründen zur Abrundung des entstandenen Gebildes, das aus den über die Lipidbrücken miteinander verbundenen Zellen besteht.

- 5 -

Zur Durchführung dieser bekannten Verfahrensweise
wird eine Kammer eingesetzt, bei der ein aus
elektrisch nicht leitenden Wänden gebildeter
Raum zur Aufnahme der die Zellen enthaltenden
Suspension vorgesehen ist, in den wenigstens
zwei metallische Elektroden derart hineinragen,
daß ein Bereich gebildet wird, in welchem die
Zellen einem zwischen den Elektroden ausgebildeten
elektrischen Feld ausgesetzt sind. Die Elektroden
sind dabei zur Erzeugung des elektrischen Durchbruches an eine Einrichtung zur Erzeugung elektrischer Spannungspulse angeschlossen. Das bekannte
Verfahren ist zu seiner Durchführung somit an
eine Kammer mit Metallelektroden und eine hieran
anschließbare Einrichtung gebunden.

Es ist Aufgabe der Erfindung, ein Verfahren
zu schaffen, das eine Fusion von Zellen mittels
des elektrischen Durchbruches ohne Verwendung
der bisher üblichen Vorrichtungen mit Metallelektroden erlaubt. Es ist ferner Aufgabe der
Erfindung, eine Vorrichtung zur Durchführung
des Verfahrens zu schaffen.

Diese Aufgabe wird gemäß der Erfindung dadurch
gelöst, daß das elektrische Feld mittels eines
als Sender ausgebildeten elektrischen Pulsgenerators
erzeugt und die elektrische Energie drahtlos
auf den Fusionsraum übertragen wird, wobei zur
Aufnahme der elektrischen Energie im Fusionsraum
diesen begrenzende Elektroden vorgesehen sind,
die aus einer Substanz bestehen, die eine gegenüber der Zellsuspension im Fusionsraum höhere Leitfähigkeit, mindestens aber $10^{-3}$ S/cm aufweist.

Das Verfahren gemäß der Erfindung ist zwar auch mit einer Kammer mit Metallelektroden durchführbar. Es bedarf jedoch einer derartigen Vorrichtung nicht. Denn durch die drahtlose Übertragung des elektrischen Feldes mittels elektromagnetischer Wellen ist das Verfahren gemäß der Erfindung nicht an das Vorhandensein einer Elektrode mit hoher Leitfähigkeit gebunden.

Bei einer Verfahrensvariante des Verfahrens gemäß der Erfindung kann so elektrische Energie über als Elektroden wirkende Lösungsschichten oder Lösungsfäden übertragen werden, wobei die die Schichten oder Fäden bildende Substanz eine wäßrige Lösung ist. Dabei können selbstverständlich auch eine aus einer Lösung bestehende Elektrode zusammen mit einer Metallelektrode vorgesehen sein.

Die Verfahrensweise gemäß der Erfindung ergibt die Möglichkeit, an die die Zellen enthaltende Suspensionslösung, die den Fusionsraum ausfüllt und eine Leitfähigkeit von nicht mehr als $10^{-2}$ S/cm aufweisen soll, eine Lösung angrenzen zu lassen, die im Hinblick auf die Verträglichkeit für die Zellen gewählt worden ist. So kann es zweckmäßig sein, daß die an den Fusionsraum angrenzende, die Lösungsschicht oder -schichten oder die Lösungsfäden bildende Lösung eine für die Zellen isotone Salzlösung ist. Diese kann beispielsweise eine Nährlösung sein, die direkt nach erfolgter

- 7 -

Fusion mit der die Zellen enthaltenden Suspensionslösung vermischt wird.

Es kann jedoch, insbesondere wenn sich die Zellen in strömender Suspensionslösung befinden und auch die die Lösungsschichten oder die Lösungsfäden bildende Lösung strömt, zweckmäßig sein, daß die elektrische Energie über Lösungsschichten oder Lösungsfäden übertragen wird, die von der Zellsuspension im Fusionsraum durch elektrisch nicht leitende Trennschichten, wie Kunststoffolien o.dgl., abgegrenzt ist. Ist dabei eine Zufuhr von Stoffen in die Suspensionslösung angestrebt, dann sind semipermeable Trennschichten zweckmäßig.

Das gegenseitige Annähern der Zellen vor dem Vorgang der Fusion im elektrischen Feld kann auf verschiedene Weise erfolgen. So können beispielsweise nach einem bisher nicht bekannten Vorschlag (Patentanmeldung 33 21 238.4) die Zellen mit magnetischen Teilchen dotiert und einem den Fusionsraum durchdringenden inhomogenen Magnetfeld derart ausgesetzt werden, daß sich die dotierten Zellen in geringem Abstand zueinander ansammeln. Da bei dieser Vorgehensweise kein elektrischer Strom fließt und somit eine für die Zellen unerwünschte Temperaturerhöhung nicht eintritt, können die Zellen dabei in einer Lösung höherer Leitfähigkeit gesammelt werden, als dies bei Anwendung eines elektrischen Feldes zur Sammlung der Zellen der Fall ist. Die Sammlung im magnetischen Feld erfordert jedoch die Dotierung der Zellen mit magnetischen Teilchen.

- 8 -

Es kann daher, wenn hiervon abgesehen werden soll, zweckmäßig sein, daß die Zellen, bevor sie dem den elektrischen Durchbruch bewirkenden elektrischen Feldpuls ausgesetzt werden, einem ebenfalls drahtlos übertragenen elektrischen Feld einer Frequenz von 1 kHz bis 100 MHz, bevorzugt 5 kHz bis 5 MHz, und einer Stärke von 10 V/cm bis 2000 V/cm je nach Größe der Zellen derart ausgesetzt werden, daß sich die Zellen in vorbestimmter Ausrichtung aneinanderreihen.

Die Verfahrensweise des Aneinanderreihens von Zellen im elektrischen Feld ist aus Biochimica et Biophysica Acta, 694 (1982), 227 - 277 (Electric Field-Mediated Fusion And Related Electrical Phenomena, U. Zimmermann) bekannt. Bei diesem bekannten Verfahren - dessen Ablauf ebenfalls unter dem Mikroskop beobachtet werden kann - wird der Membrankontakt zwischen wenigstens zwei Zellen durch Anlegen eines alternierenden, im allgemeinen schwach inhomogenen Feldes herbeigeführt. Durch das elektrische Feld werden, bedingt durch Polarisationsprozesse in der Zelle, Dipole erzeugt, die sich gegenseitig anziehen, wenn sich die Zellen während ihrer Wanderung im elektrischen Feld einander nähern (sogenannte Dielektrophorese).

Nach der Bildung der Zellenreihe werden die Störungen in der Membranstruktur zwischen benachbarten Zellen durch den elektrischen Durchbruchpuls ausgelöst.

- 9 -

Zur Durchführung des Verfahrens gemäß der Erfindung ist eine Vorrichtung geeignet, bei der ein aus elektrisch nichtleitenden Wänden gebildeter Raum zur Aufnahme der die Zellen enthaltenden Suspension und wenigstens zwei derart angeordnete Elektroden vorgesehen sind, daß ein zwischen den Elektroden liegender Bereich gebildet wird, in welchem die Zellen einem zwischen den Elektroden ausgebildeten elektrischen Feld ausgesetzt sind, und bei der eine Einrichtung zur Erzeugung eines pulsweisen elektrischen Feldes mit einer $10^3$ bis $10^5$ V/cm betragenden Feldstärke vorgesehen ist. Bei der Vorrichtung gemäß der Erfindung ist die Einrichtung zur Erzeugung des elektrischen Pulses als Sender ausgebildet.

Bei hinreichend starkem Sender ist eine Anpassung der Elektroden als Empfänger nicht erforderlich. Es kann jedoch zweckmäßig sein, daß die Elektroden als Empfänger ausgebildet sind. Dabei kann eine der Elektroden geerdet und die andere Elektrode mit einer Antenne versehen sein, die - soweit möglich - auf den Frequenzbereich der zu empfangenden elektrischen Signale abgestimmt ist.

Bei einer Ausführungsform der Vorrichtung gemäß der Erfindung bestehen die Elektroden aus Metall, beispielsweise Platin, die in den Fusionsraum hineinragen.

Eine weitere Ausführungsform der Vorrichtung gemäß der Erfindung besteht darin, daß zur Bildung von aus Lösung bestehenden Elektroden dünne, die Elektroden gegenüber dem zur Aufnahme der Zellsuspension vorgesehenen Raum abgrenzende Trennschichten aus elektrisch nicht leitendem Material vorgesehen sind.

Die die Elektroden abgrenzenden Trennschichten können als Röhrchen ausgebildet und in dem zur Aufnahme der die Zellen enthaltenden Raum angeordnet sein.

Für den Fall, daß eine kontinuierliche Zufuhr von Zellsuspension in den Fusionsraum vorgesehen ist, ist zweckmäßigerweise der zur Aufnahme der Zellsuspension vorgesehene Raum als Durchflußraum ausgebildet. Dies ergibt beispielsweise auch die Möglichkeit, Zellsuspensionslösung schubweise in den Fusionsraum einzugeben, wobei während des eigentlichen Fusionsvorganges der Durchfluß gestoppt ist. Desgleichen können die Räume, die zur Bildung von aus Lösung bestehenden Elektroden durch die Trennschichten gegenüber dem zur Aufnahme der Zellsuspension vorgesehenen Raum abgegrenzt sind, als Durchflußräume ausgebildet sein.

Um zu ermöglichen, daß Stoffe aus der die Elektroden bildenden Lösung in den die Zellen enthaltenden Fusionsraum gelangen - was für die weitere Behandlung der Zellen wichtig sein kann - sind als Trennschichten semipermeable Schichten vorgesehen. Als röhrchenartige Trennschichten können dabei Dialysier-Schläuche

0133978

oder dünne poröse Glaskapillaren verwendet
werden.

Für den Fall, daß die Zellen vor der Fusion
durch Dielektrophorese aneinandergereiht und
somit in Kontakt zueinander gebracht werden
sollen, ist außerdem eine weitere, als Sender
ausgebildete Einrichtung zur Erzeugung eines
elektrischen Feldes einer Frequenz im Bereich
von 1 kHz bis 100 MHz, bevorzugt von 5 kHz
bis 5 MHz, und einer Stärke im Bereich von
10 V/cm bis 2000 V/cm vorgesehen.

In der Zeichnung sind Ausführungsbeispiele
der Vorrichtung gemäß der Erfindung schematisch
dargestellt und werden im folgenden näher
erläutert.

Es zeigen

Figur 1    eine als Sender ausgebildete Einrichtung
           mit als Empfänger wirkender Elektrodenanordnung,

Figur 2    Vorrichtung gemäß Figur 1 als elektrisches Ersatzschaltbild,

Figur 3    Fusionskammer mit den Fusionsraum
           als Elektrode begrenzenden Lösungsschichten,

Figur 4    Fusionskammer mit in den Fusionsraum hineinragenden, als Elektroden
           wirkenden Lösungsfäden,

Figur 5    Fusionskammer mit Kombination einer
           Metallelektrode mit einem als Elektrode
           wirkenden Lösungsfaden.

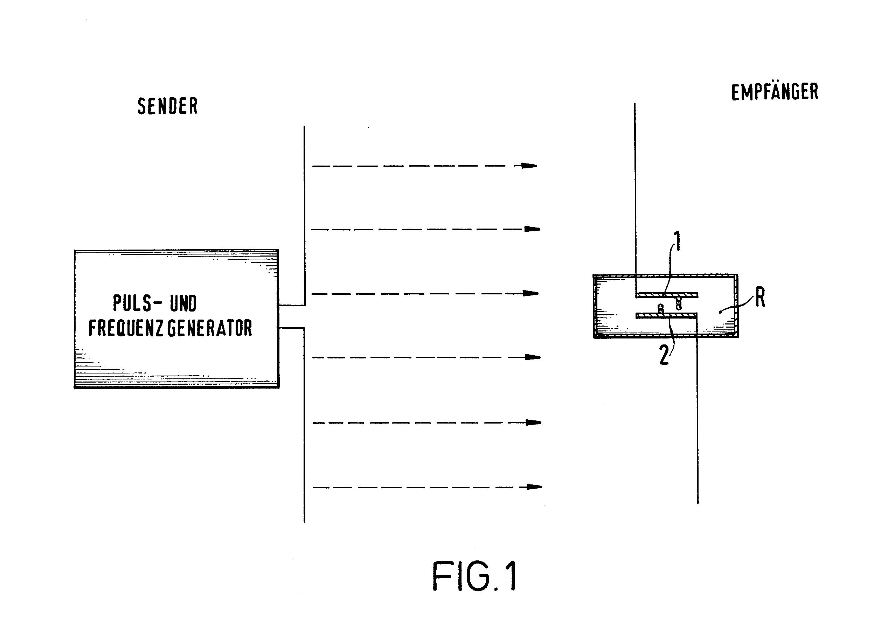

Aus Figur 1 geht die prinzipielle Anordnung des als Sender ausgebildeten Puls- und Frequenzgenerators und der als Empfänger vorgesehenen
Fusionskammer hervor. Die schematische Darstellung zeigt einen Raum R zur Aufnahme der
die Zellen enthaltende Suspension, in den
zwei Elektroden 1 und 2 - in diesem Falle
zwei Metallelektroden - hineinragen. Die Elektroden sind jeweils mit nach außen führenden
und als Antenne wirkenden Kabeln verbunden.
Zellen, die sich in dem zwischen den Elektroden
liegenden Bereich befinden, werden einem elektrischen Feld ausgesetzt, das durch drahtlose
Übertragung vom Sender auf den Empfänger erzeugt
worden ist.

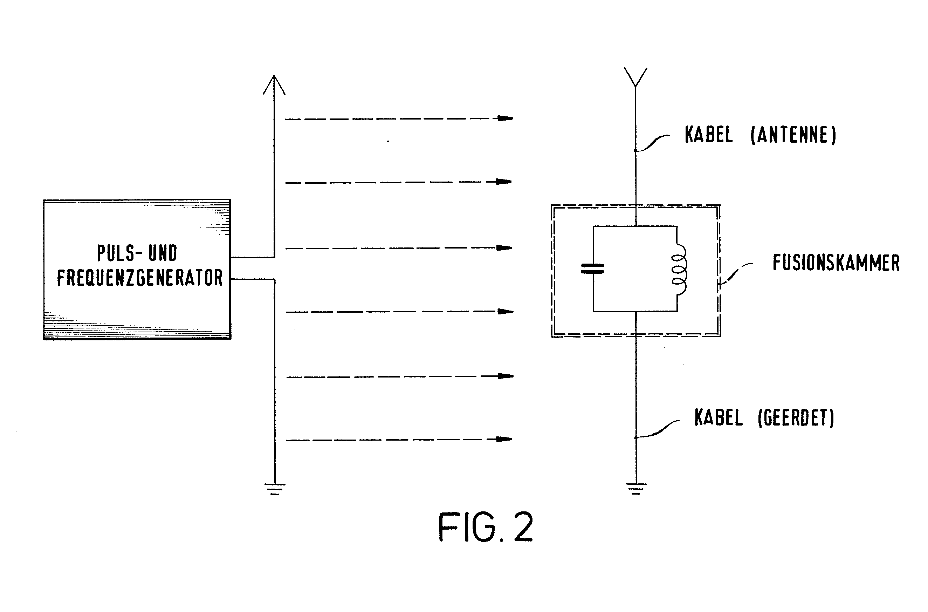

In Figur 2 ist die in Figur 1 dargestellte
Anordnung in anderer Darstellung - die Fusionskammer in Form eines elektrischen Ersatzschaltbildes für einen elektrischen Schwingkreis -
wiedergegeben.

Mit einer Vorrichtung der in Figur 1 beschriebenen Art (Elektroden aus Platindraht; Elektrodendicke 300 µm; Elektrodenlänge ca. 2 cm; Elektrodenabstand 200 µm; Länge der Kabel ca 40 cm,
ein Kabel geerdet) wurden Avena-sativa-Protoplasten in einer wäßrigen 0,5 M Mannit-Lösung
zunächst in einem drahtlos übermittelten Wechselfeld einer Frequenz von 1 MHz und einer Intensität
von 200 V/cm aneinandergereiht.

Die aneinandergereihten Zellen wurden sodann
durch einen ebenfalls drahtlos übermittelten
Feldpuls von 20 µs Dauer und einer Intensität
von 750 V/cm fusioniert.

Der Abstand zwischen Sender und Empfänger betrug dabei etwa 3 m.

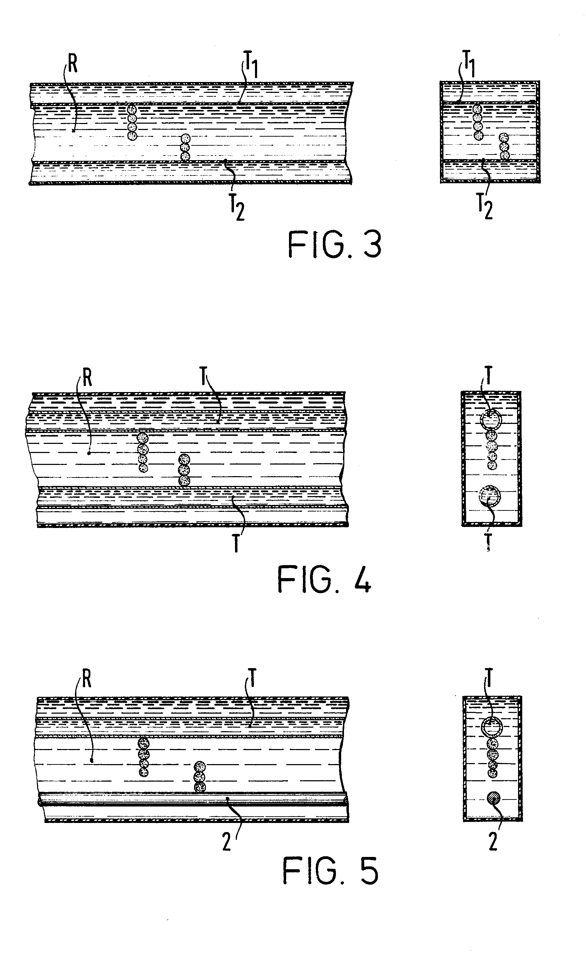

In Figur 3 ist ein Teil einer Fusionskammer wiedergegeben, die durch elektrisch nicht leitende Wände begrenzt ist und innerhalb deren Trennschichten $T_1$ und $T_2$ aus Polyethylen vorgesehen sind. Durch diese Trennschichten werden als Elektroden wirkende Flüssigkeitsschichten vom eigentlichen Fusionsraum abgegrenzt.

Figur 4 zeigt eine Ausführungsform der Fusionskammer, in welche röhrchenartig ausgebildete Trennschichten T hineinragen, beispielsweise Glaskapillaren von 450 µm Außendurchmesser und 340 µm Innendurchmesser. Die in den Röhrchen befindlichen Flüssigkeiten (z.B. eine KCl-Lösung; Konzentration C = 100 mM; Leitfähigkeit: G = $10^{-2}$ S/cm) wirken als Elektroden, zwischen denen Zellenreihen gebildet und die aneinanderliegenden Zellen fusioniert werden.

Bei der in Figur 5 dargestellten Fusionskammer besteht eine der Elektroden, die Elektrode 2, aus einem Metalldraht. Die andere Elektrode wird dagegen durch eine innerhalb des Röhrchens befindliche Lösung gebildet.

Mit einer Vorrichtung der in Figur 4 dargestellten Art wurden Vicia-faba-Protoplasten in einer wäßrigen 0,5 M Mannit-Lösung fusioniert. Die Glaskapillaren hatten eine Länge von etwa 2 cm. Sie waren mittels einer KCl-Leitfähigkeitspaste elektrisch mit ca. 40 cm langen Kabeln verbunden.

Kernforschungsanlage Jülich
Gesellschaft mit beschränkter Haftung

0133978

P a t e n t a n s p r ü c h e

1. Verfahren zur Fusion von Zellen, bei dem in einer Zellsuspension im Fusionsraum befindliche Zellen auf einen geringen Abstand zueinander gebracht werden und die Zellen dem Puls eines elektrischen Feldes einer solchen Stärke ausgesetzt werden, daß die Membran der Zellen durchbricht, wodurch bei benachbarten Zellen Störungen in der Membranstruktur verursacht werden, die zur Bildung von Membranbrücken zwischen den benachbarten Zellen und zur Verschmelzung der Zellen führen, d a d u r c h g e k e n n z e i c h n e t , daß das elektrische Feld mittels eines als Sender ausgebildeten elektrischen Pulsgenerators erzeugt und die elektrische Energie drahtlos auf den Fusionsraum übertragen wird, wobei zur Aufnahme der elektrischen Energie im Fusionsraum diesen begrenzende Elektroden vorgesehen sind, die aus einer Substanz bestehen, die eine gegenüber der Zellsuspension im Fusionsraum höhere Leitfähigkeit, mindestens aber $10^{-3}$ S/cm aufweist.

2. Verfahren nach Anspruch 1, d a d u r c h g e k e n n z e i c h n e t , daß die elektrische Energie über als Elektroden wirkende Lösungsschichten oder Lösungsfäden übertragen wird, wobei die die Schichten oder Fäden bildende Substanz eine wäßrige Lösung ist.

3. Verfahren nach Anspruch 2, d a d u r c h
g e k e n n z e i c h n e t , daß die die
Lösungsschicht oder den Lösungsfaden bildende
Lösung eine für die Zellen isotone Salzlösung
ist.

4. Verfahren nach einem der Ansprüche 1 bis 3,
d a d u r c h   g e k e n n z e i c h n e t ,
daß die elektrische Energie über Lösungsschichten
oder Lösungsfäden übertragen wird, die von
der Zellsuspension im Fusionsraum durch elektrisch
nichtleitende Trennschichten, wie Kunststofffolien o.dgl., abgegrenzt sind.

5. Verfahren nach Anspruch 4, d a d u r c h
g e k e n n z e i c h n e t , daß die Trennschichten semipermeabel sind.

6. Verfahren nach einem der Ansprüche 1 bis 5,
d a d u r c h   g e k e n n z e i c h n e t ,
daß die Zellen, bevor sie dem oder den den
elektrischen Durchbruch bewirkenden elektrischen
Feldpuls ausgesetzt werden, einem ebenfalls
drahtlos übertragenen elektrischen Feld einer
Frequenz von 1 kHz bis 100 MHz, bevorzugt
5 kHz bis 5 MHz und einer Stärke von 10 V/cm
bis 2000 V/cm je nach Größe der Zellen derart
ausgesetzt werden, daß sich die Zellen in
vorbestimmter Ausrichtung aneinanderreihen.

7. Vorrichtung zur Durchführung des Verfahrens
nach einem der Ansprüche 1 bis 6, bei der
ein aus elektrisch nichtleitenden Wänden gebildeter Raum zur Aufnahme der die Zellen

- 3 -

enthaltenden Suspension und wenigstens zwei
derart angeordnete Elektroden vorgesehen sind,
daß ein zwischen den Elektroden liegender
Bereich gebildet wird, in welchem die Zellen
einem zwischen den Elektroden ausgebildeten
elektrischen Feld ausgesetzt sind, und bei
der eine Einrichtung zur Erzeugung eines pulsweisen
elektrischen Feldes mit einer $10^3$ bis $10^5$ V/cm
betragenden Feldstärke vorgesehen ist, d a -
d u r c h   g e k e n n z e i c h n e t , daß
die Einrichtung zur Erzeugung des elektrischen
Pulses als Sender ausgebildet ist.

8. Vorrichtung nach Anspruch 7, d a d u r c h
g e k e n n z e i c h n e t , daß die Elektroden
als Empfänger ausgebildet sind.

9. Vorrichtung nach Anspruch 7 oder 8, d a d u r c h
g e k e n n z e i c h n e t , daß die Elektroden
aus Metall, beispielsweise aus Platin, bestehen
und in den zur Aufnahme der die Zellen enthaltenden
Suspension vorgesehenen Raum hineinragen.

10. Vorrichtung nach Anspruch 7, d a d u r c h
g e k e n n z e i c h n e t , daß zur Bildung
von aus Lösung bestehenden Elektroden dünne,
die Elektroden gegenüber dem zur Aufnahme
der Zellsuspension vorgesehenen Raum abgrenzende Trennschichten aus elektrisch nicht leitendem
Material vorgesehen sind.

11. Vorrichtung nach Anspruch 10, d a d u r c h
g e k e n n z e i c h n e t , daß die die
Elektroden abgrenzenden Trennschichten röhrchenartig ausgebildet und in dem zur Aufnahme

- 4 -

der Zellsuspension vorgesehenen Raum angeordnet
sind.

12. Vorrichtung nach einem der Ansprüche 7 bis 11,
d a d u r c h   g e k e n n z e i c h n e t ,
daß der zur Aufnahme der Zellsuspension vorgesehene Raum als Durchflußraum ausgebildet
ist.

13. Vorrichtung nach einem der Ansprüche 7 bis
12, d a d u r c h   g e k e n n z e i c h -
n e t , daß die Räume, die zur Bildung von
aus Lösung bestehenden Elektroden durch die
Trennschichten gegenüber dem zur Aufnahme
der Zellsuspension vorgesehenen Raum abgegrenzt
sind, als Durchflußräume ausgebildet sind.

14. Vorrichtung nach einem der Ansprüche 10 bis
13, d a d u r c h   g e k e n n z e i c h -
n e t , daß die Trennschichten semipermeabel
sind.

15. Vorrichtung nach einem der Ansprüche 7 bis
14, d a d u r c h   g e k e n n z e i c h -
n e t , daß eine weitere, als Sender ausgebildete
Einrichtung zur Erzeugung eines elektrischen
Feldes einer Frequenz im Bereich von 1 kHz
bis 100 MHz, bevorzugt von 5 kHz bis 5 MHz,
und einer Stärke im Bereich von 10 V/cm bis
2000 V/cm vorgesehen ist.

SENDER

EMPFÄNGER

PULS- UND
FREQUENZ GENERATOR

1

R

2

FIG.1

1/3

0133978

FIG. 2

PULS- UND
FREQUENZGENERATOR

KABEL (ANTENNE)

FUSIONSKAMMER

KABEL (GEERDET)

2/3

0133978

FIG. 3

FIG. 4

FIG. 5